# EUROPEAN PATENT APPLICATION

(11) **EP 4 282 402 A1**
(43) Date of publication of application: **29.11.2023**
(21) Application number: 21920749.5
(22) Date of filing: 29.11.2021
(51) Int. Cl.: A61K 9/08, A61K 9/66, A61K 47/10, A61K 47/26, A61K 47/58, A61K 47/36, A61K 47/38, A61K 31/573, A61K 31/405, A61P 27/02, A61P 27/10

(54) **OPHTHALMIC PREPARATION FOR TREATING MACULAR EDEMA, OPTIC NEURITIS AND NON-INFECTIOUS ENDOPHTHALMITIS THROUGH EYE DROP ADMINISTRATION**

(30) Priority: 22.01.2021 CN 202110091088
(71) Applicant: Chengdu Ruimu Biopharmaceuticals Co., Ltd., Chengdu, Sichuan 611135 (CN)
(72) Inventor: DONG, Qing, Chengdu, Sichuan 611135 (CN); XUE, Lubing, Chengdu, Sichuan 611135 (CN)
(74) Representative: Altmann Stößel Dick Patentanwälte PartG mbB
(86) International application number: PCT/CN2021/134151
(87) International publication number: WO 2022/156373

(57) **Abstract**

An ophthalmic preparation for treating macular edema, optic neuritis and non-infectious endophthalmitis. The ophthalmic preparation is a preparation composed of an active ingredient for treating eye diseases and an ophthalmic preparation carrier or auxiliary material; the active ingredient for treating eye diseases is a glucocorticoid medicine and/or a non-steroidal medicine; the ophthalmic preparation carrier or auxiliary material comprises the following ingredients: a surfactant, an ionic polymer and a solvent, or the ophthalmic preparation carrier or auxiliary material contains the following ingredients: povidone with low polymerization degree, povidone with moderate polymerization degree and a solvent. The ophthalmic preparation can carry (wrap) the glucocorticoid and/or the non-steroidal medicines to penetrate through the anterior segment of eyes and be conveyed to the posterior segment of eyes to treat the macular edema, the optic neuritis and the non-infectious endophthalmitis in an eye drop administration mode, and the ophthalmic preparation has extremely excellent clinical use value and positive social significance.

## Description

### Technical field

The present invention belongs to the field of ophthalmic medicaments, and in particular relates to an ophthalmic preparation for treating macular edema, optic neuritis and non-infectious endophthalmitis by eye drop administration.

### Background technology

There are many patients with fundus diseases, and only in China, the number of patients has exceeded tens of millions. With the aging society and the popularity of electronic products, the incidence rate will increase year by year; common fundus diseases include diabetic macular edema, diabetic retinopathy (DR), age-related macular diseases, retinal vein occlusion (RVO), pathological myopia, geographic atrophy, eye tumors, endophthalmitis, etc., which may lead to decreased vision or even blindness, seriously affecting people's quality of life. For example, about 6.8% of diabetics suffer from diabetic macular edema (DME), which is the main cause of blindness in diabetics (Urias et al., Vision Research, V139:221-227, 2017; Mandal et al., Ocular delivery of proteins and peptides: Challenges and novel formulation approaches, Advanced Drug Delivery Reviews, 126:67-95, 2018).

Drug therapy is the main treatment method and research trend for fundus diseases. However, because of the complex physiological structure and barrier in the eyes, it is very difficult for medicaments to enter the eyeball, especially the posterior segment of the eye, and thus it is hard to achieve the effective dose, thereby, that brings great difficulties in realizing an effective therapy. Finding an effective and safe treatment for fundus diseases is the object that researchers in this field have been striving for.

There are usually three ways for clinical ophthalmic administration: (1) Conjunctival sac administration (eye drops): medicaments can diffuse and distribute to the iris and Ciliary body after entering the anterior chamber water through the cornea, but the barrier effect of the lens and vitreous membrane makes it difficult for drugs to enter the lens and vitreous body; (2) Eye injection administration: it includes subconjunctival injection, anterior chamber injection, vitreous injection, retroocular injection, and orbital injection. Injecting medication can make the medicament directly reach the treatment site, but injection is traumatic and poses potential risks, such as anterior chamber injection can cause pain, photophobia, tearing, anterior chamber turbidity, bleeding, corneal endothelial cell damage, traumatic cataracts, etc; vitreous injection can cause lens opacity, vitreous organization, retinal/optic nerve damage, etc; (3) Systemic administration includes oral administration and intravenous administration: medicaments in the body generally gather in the liver, kidney or lung, and are blocked by the blood retinal barrier (BRB), so the concentration reaching the eye tissue is lower. Meanwhile, the whole body, especially the main organs, suffer unnecessary side effects.

At present, in order to allow medicaments to pass through the eye barrier in clinical practice, technical means such as eyeball intravitreal injections (IVI) or implant (ophthalmic insert) are usually used to deliver medicaments to the vitreous body of the patients for treating posterior segment diseases (Pei-Xue Ling, "Ophthalmic Drugs and Preparation Techniques", China Light Industry Press, 2010, P3; Wang et al., Mediators of Inflammation, Vol. 2013, Article ID 780634; Luaces-Rodríguez et al., Pharmaceutics, 2018, 10, 66). The operation for intravitreal injection or implant of medicaments is traumatic, that requires specially trained ophthalmologists to perform in sterile environments such as operating rooms; due to the traumatic nature of the operation, complications may occur, such as high intraocular pressure, cataracts, iatrogenic infectious endophthalmitis, vitreous hemorrhage, retinal damage, etc; the requirements of operating conditions and environment are high, and the operation must be carried out in hospitals where the conditions are permitted; the production and usage costs of biopharmaceutical eye injections are high; at the same time, for treatment opportunity, there are also cases of delayed treatment due to medical conditions, resulting in poor flexibility in adjusting dosage regimen (M. HATA et al., RETINA, 37: 1320-1328, 2017).

In fact, the current ophthalmic injections of biopharmaceutics origin, which are used to treat wet age-related macular degeneration (wAMD) and Diabetes-related macular Edema (DME) and the same, are administered by intravitreal injections. According to the drug instructions, they need to be injected once every 1-3 months, and long-term injection is required. In addition, the glucocorticoid dexamethasone has also been developed as an intravitreal implant (ophthalmic insert, trade name: Ozurdex^{®}), and is used to treat macular edema caused by retinal vein occlusion in the posterior segment of the eye, and its efficacy can last for 6 months after implant. But after implant, the intraocular pressure of some patients increased and reached a peak value after administration for two months (Ozurdex^{®} Patient Management Manual, Allergan, CN/011/2018), and thus there is a risk of side effects.

Conjunctival sac administration is the most convenient and safe way of eye medication. However, the cornea has a multi-layer structure, that is roughly divided from the outside to the inside into: an epithelial layer rich in liposomes, a stromal layer rich in water-based components, and an endothelial layer rich in liposomes. After eye drops, the eye drops first contact with the tear layer on the surface of the eye, and then need to cross the epithelial layer, stromal layer, and endothelial layer to reach the posterior segment of the eye. During this process, due to the dilution of tears, the ocular surface barrier of the cornea and conjunctiva, and the anatomical position of the lens and vitreous body, eye drops often have high concentrations in the tissues of the anterior segment, and it is difficult for the eye drop to enter the posterior segment and achieve effective therapeutic concentrations. Therefore, although the way of conjunctival sac administration is safe, its drug delivery is poor, and thus brings great difficulties in achieving the object of effectively treating fundus diseases.

To sum up, the main drug delivery ways in the prior art for the treatment of fundus diseases such as macular edema are difficult to give consideration to both safety and effectiveness.

### Summary of the invention

Compared with intravenous injection and intravitreal injection, the way of eye drop administration has significant advantages in safety and convenience. Inventing ophthalmic preparations that can deliver drugs to the posterior segment of the eye to treat macular edema is a technical problem that needs to be solved urgently in clinical practice, which is of great clinical treatment value and social significance.

The object of the present invention is to provide an ophthalmic medicament for eye drops, which can deliver the active pharmaceutical ingredients for treating eye diseases to the posterior segment of the eye to treat macular edema, optic neuritis, and non-infectious endophthalmitis.

The present invention provides an ophthalmic preparation for eye drops, which is a preparation composed of an active pharmaceutical ingredient for treating eye diseases as well as an ophthalmic preparation carrier or auxiliary material;
the active ingredient for treating eye diseases is a glucocorticoid medicine and/or a non-steroidal medicine;
the ophthalmic preparation carrier or auxiliary material comprises the following ingredients: a surfactant, an ionic polymer and a solvent;
or, the ophthalmic preparation carrier or auxiliary material contains the following ingredients: povidone with low polymerization degree, povidone with medium polymerization degree and a solvent.

Further, in the forementioned ophthalmic preparation carrier or auxiliary material, the mass ratio of the surfactants to the ionic polymer is: (1-100):(0.1-50); the ratio of the surfactant to the solvent is: every 100 mL of the solvent contains 5-3000 mg of the surfactant.

More further, in the forementioned ophthalmic preparation carrier or auxiliary material, the mass ratio of the surfactants to the ionic polymer is (12-31):(2-7.5); the ratio of the surfactant to the solvent is: every 100 mL of the solvent contains 880-1240 mg of the surfactant;
further, the above-mentioned surfactant is a non-ionic surfactant; preferably, the non-ionic surfactant is Spans, Polysorbates, Poloxamer, alkylglucosides, vitamin E polyethylene glycol succinate (TPGS), sucrose stearates or azone; Spans or polysorbates are preferable.

Further, the ionic polymer mentioned above is selected from at least one of carboxymethyl cellulose (CMC) and its salts, sodium starch glycolate, hyaluronic acid and its salts, Xanthan gum, alginic acid and its salts, and polyethylene glycol diacetate PEG-(COOH)₂; preferably, the ionic polymer is selected from at least one of carboxymethyl cellulose and its salts, hyaluronic acid and its salts.

Further, in the above-mentioned ophthalmic preparation carrier or auxiliary material, the mass ratio of the povidone with low polymerization degree to the povidone with medium polymerization degree is (0.1-10):1, and the ratio of the povidone with low polymerization degree to the solvent is: every 100 mL of solvent contains 5-3000 mg of the povidone with low polymerization degree;
preferably, the mass ratio of the povidone with low polymerization degree to the povidone with medium polymerization degree is (0.24-0.8):1, and the ratio of the povidone with low polymerization degree to the solvent is: every 100 mL of solvent contains 240-840 mg of the povidone with low polymerization degree.

More further, the above-mentioned povidone with low polymerization degree is a povidone with a weight average molecular weight of 2000-5000, and preferably is a povidone PVP K12 with a weight average molecular weight of 3500.

More further, the povidone with medium polymerization degree is a povidone with a weight average molecular weight of 20000-60000, and preferably is a povidone PVP K30 with a weight average molecular weight of 35000-50000.

Further, in the above-mentioned ophthalmic preparation carrier or auxiliary material, the solvent is a polar solvent, and preferably water.

Further, the above-mentioned ophthalmic preparation carrier or auxiliary material also contains the following components: adhesive agents and/or cosolvents;
more further, the adhesive agent is selected from at least one of polyethylene glycol, carbomer, Poloxamer, povidone, hydroxypropyl cellulose, methyl cellulose, hydroxyethyl cellulose, polyvinyl alcohol, Xanthan gum, polyoxyethylene fatty alcohols, hyaluronic acid and its salts or hydroxypropyl methyl cellulose (HPMC); the cosolvent is propylene glycol, glycerol, liquid polyethylene glycol or castor oil; the mass ratio of the adhesive agent to the surfactant or the adhesive agent to the povidone with low polymerization degree is 1:(0.1-100), and the mass ratio of the cosolvent to the surfactant or the cosolvent to the povidone with low polymerization degree is (1-10): 1;
more preferably, the mass ratio of the adhesive agent to the surfactant is 1:(1.2-30), and the mass ratio of the cosolvent to the surfactant is (2.56-9): 1;
further, the mass ratio of the surfactant or the povidone with low polymerization degree to the active pharmaceutical ingredient for treating eye diseases is (12-31): 1.

Further, the above-mentioned glucocorticoid medicament is at least one of dexamethasone, hydrocortisone, prednisolone and betamethasone; the non-steroidal medicament is at least one of diclofenac, pranoprofen, indomethacin and bromfenac sodium.

Further, the above-mentioned ophthalmic preparation carrier or auxiliary material contains nanobodies, which are formed by self-assembly of the components of the ophthalmic preparation carrier or auxiliary material; the nanobodies are assembled with active pharmaceutical ingredients for treating eye diseases.

More further, the above-mentioned nanobody is spherical, with a particle size of 1-100 nm; preferably, the particle size of the nanobody is 5-30 nm.

More further, the above-mentioned preparation contains nanospheres, which are spherical in shape and have a particle size of 10-2000 nm. The nanospheres are formed by self-assembly of nanobodies; preferably, the particle size of the nanospheres is 100-2000 nm.

The present invention also provides the method for preparing the above-mentioned preparation, which comprises the following steps:
(1) The surfactant and/or the adhesive agent is added to the solvent to prepare a solution;
(2) The active pharmaceutical ingredient for treating eye diseases and/or the cosolvent is dispersed in the solution obtained in step (1), to which is then added the ionic polymer or its solution, and the resultant solution is dispersed and mixed to obtain the initial suspension;
(3) The initial suspension obtained in step (2) is stirred and dispersed or homogenized to obtain the preparation;
or comprises the following steps:
(a) The povidone with low polymerization degree and/or the adhesive agent is added to the solvent to prepare a solution;
(b) The active pharmaceutical ingredient for treating eye diseases and/or the cosolvent is dispersed in the solution obtained in step (a), to which is then added the povidone with medium polymerization degree or its solution, and the resultant solution is dispersed and mixed to obtain the initial suspension;
(c) The mixed solution obtained in step (b) is ground or uniformly dispersed to obtain the preparation.

Further, the dispersion described in step (2) or step (b) is selected from at least one of mechanical stirring dispersion, magnetic stirring dispersion, vortex shaking dispersion, shear dispersion, homogeneous dispersion, grinding dispersion, and ultrasonic dispersion.

The present invention also provides the use of the preparation mentioned above in the preparation of medicaments for treating fundus diseases; preferably, the medicament for treating fundus diseases is a medicament for treating macular edema, and/or optic neuritis, and/or non-infectious endophthalmitis.

Further, the medicament for treating macular edema is a medicament for treating macular edema caused by fundus vascular diseases, macular edema caused by central retinal vein occlusion, macular edema caused by branch retinal vein occlusion, diabetic macular edema (DME), pathological myopic macular edema, and/or macular edema caused by wet age-related macular degeneration.

The present invention also provides a method for treating fundus diseases, that is, the preparation mentioned above is administrated to the patients.

Further, the fundus disease is macular edema, and/or optic neuritis, and/or non-infectious endophthalmitis.

More further, the above-mentioned macular edema is macular edema caused by fundus vascular diseases, macular edema caused by central retinal vein occlusion, macular edema caused by branch retinal vein occlusion, diabetic macular edema (DME), pathological myopic macular edema, and/or macular edema caused by wet age-related macular degeneration.

More further, the way used is eye drop administration.

The experimental results have shown that the ophthalmic preparation for eye drop administration prepared in the present invention has stable properties and is easy to store; and can effectively deliver the active pharmaceutical ingredients for treating eye diseases to the posterior segment of the eye; reach an effective (expected) concentration in the fundus; achieve the treatment of fundus diseases such as macular edema; overcome the problems of current intravitreal injection, intravitreal injection implant, oral administration and systemic injection; solve the serious complications problems such as intraocular hemorrhage and pain; greatly reduce the pain of patients with fundus diseases; increase medical compliance; improve the life quality of patients and their families; or avoid systemic toxic and side effects caused by systemic medication.

The present invention can avoid complications caused by local injection or implant of the eye.

The preparation developed in the present invention has a small dosage as well as minimal toxic and side effects, and can not only be used as a therapeutic medicament, but also as a control and prevention for ophthalmic diseases.

The preparation of the present invention can meet the needs of long-term administration in clinical practice.

In the eye drop medication system of the present invention, the active pharmaceutical ingredient can be a small molecule drug that has been used in clinical practice and has a clear mechanism of action. The quality is controllable, the product is easy to use, the patient has good compliance, and the doctor can flexibly adjust the medication scheme according to the patient's conditions.

The nanobody used in the present invention refers to a nanoscale spherical aggregate formed by self-assembly of the components of the ophthalmic preparation carrier or excipient in a solvent.

The nanosphere used in the present invention refers to spherical self-assembled structures formed by the self-assembly of nanobodies in a solvent.

The solvent used in the present invention refers to a liquid that can dissolve the components of an ophthalmic preparation carrier or excipient.

The surfactant used in the present invention refers to a substance that can significantly reduce the surface tension of a solution; the non-ionic surfactant used in the present invention refers to a surfactant that does not dissociate in water.

The ionic polymer used in the present invention refers to a polymer with cations or anions.

The povidone with low polymerization degree used in the present invention refers to a povidone with a molecular weight of <10000 Dalton, while the povidone with medium polymerization degree refers to a povidone with a molecular weight of >10000 Dalton but <100000 Dalton.

The "active pharmaceutical ingredient for treating eye diseases" used in the present invention refers to an Active Pharmaceutical Ingredient (API) that can be used for treating eye diseases, and has already been used as an ophthalmic medicament, as well as the mechanism and target of action indicate that this API can treat eye diseases, but has not been used as an ophthalmic medicament yet.

The eye drop administration of the present invention refers to an administration method of dropping the drug solution into the eye, which belongs to the corneal route of administration.

Obviously, based on the above content of the present invention, according to the common technical knowledge and the conventional means in the field, without department from the above basic technical spirits, other various modifications, alternations, or changes can further be made.

With reference to the following specific examples of the embodiments, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. The transmission electron microscopy image of the sample prepared in Example 1 (with a scale of 200 nm) (A); the transmission electron microscopy image after staining with staining agent (with a scale of 1 µm) (B).

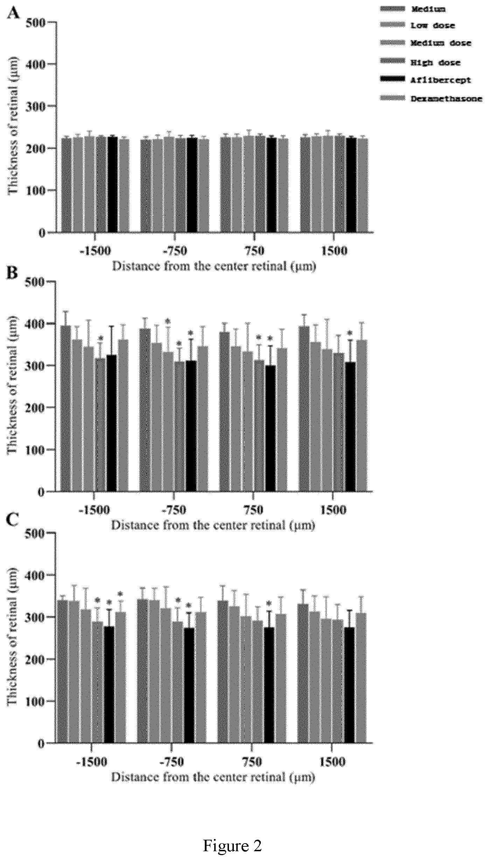

Figure 2. The left and right sides of the veins in each group of animals were respectively blocked at approximate 750 µm and 1500 µm of the thickness of the retina at different test time points. The horizontal axis represents the vertical distance between the measurement point in the OCT image and the center of the optic disc (µm); the vertical axis represents the full thickness of the retina (µm); the data is expressed as mean ± standard deviation (wherein 2A represents the full thickness of the retina in each group before modeling, N = 8; 2B represents the full thickness of the retina in each group on D2, with N=6 in the solvent control group and low-dose group, N=8 in the medium and high-dose groups, and N=7 in the dexamethasone group; 2C represents the total thickness of the retina in each group on D4, with N=6 in the solvent control group, N=7 in the test substance dose group, and N=8 in the medium and high dose groups of the test substance, Aflibercept group, and dexamethasone group; compared with the same period of solvent control group, *p ≤ 0.05).
Figure 3. Images of fundus color photography and fluorescein angiography.
Figure 4. Optical coherence tomography (measurement of retinal thickness).

### Examples

The reagents or instruments used in the present invention can be obtained from market, and if specific conditions are not specified, they shall be used according to conventional conditions or manufacturer's recommended conditions.

Some instruments and equipment are as follows:
ES225SM-DR(E) electronic analytical balance, Precisa (Switzerland);
DF-101S Heat-collection type thermostatic magnetic agitator, Gongyi Yingyu Rivet Factory (Henan, China);
WH-2 Mini Vortex meter, Shanghai Huxi Analysis Instrument Factory (Shanghai, China);
Disperse machine: T25 easy clean digital, IKA (Germany);
KQ-500 type ultrasonic cleaning machine, Kunshan Ultrasonic Instruments Co., Ltd (Kunshan, China)
JP-010T type ultrasonic cleaning machine, Skymen Cleaning Equipment Shenzhen Co., Ltd.;
AH-NANO Plus High Pressure Homogenizer, Antos Nano Technology (Suzhou) Co., Ltd. (China);
PM-DK2 Planetary Ball Mill, Droide Instrument and Equipment (Shanghai) Co., Ltd. (Shanghai, China);
Mettler Toledo FE20 pH meter, Mettler-Toledo (Switzerland);
NS-90 Nanoparticle Size Analyzer, Zhuhai Omec Instrument Co., Ltd (Zhuhai, China);
Agilent 1100 HPLC, Agilent Technologies Inc. (USA);
API4000 Triple Quadrupole Mass Spectrometer (Applied Biosystems, USA);
STY-1A Osmotic Pressure Measuring Instrument, Tiandatianfa Technology Corp., Ltd (Tianjin, China).

The method for detecting the properties of the preparation according to the present invention was as follows:
Method for measuring particle size:
1 mL of sample prepared in the Example or Comparative Example was transferred to the sample cell. The detection temperature was set to 40 °C, and then the sample cell was put into the NS-90 Nanoparticle Size Analyzer to start the detection. Each sample was tested three times, and the particle size (in terms of light intensity distribution and % percentage) and polydispersity index (PdI) were represented with the average of three test results.

Method for measuring osmotic pressure:
The osmotic pressure molar concentration of the solution was measured by measuring its freezing-point depression. Procedures: the probe of STY-1A osmotic pressure measuring instrument was cleaned. Three portions of 100 µL distilled water were respectively added to three sample tubes, and after preheating the instrument, the sample tube containing 100 µL of distilled water was screwed onto the instrument probe, followed by selecting "clean 3 times" and clicking "Clean", that was repeated three times. Measuring: After filling in the sample information in the instrument information table, click "Testing"; a pipette was used to transfer 100 µL of sample into the sample tube, which was gently screwed onto the instrument, followed by clicking "Start" for testing. The test was repeated three times, and the average of the three test results was taken as the result.

Method for measuring pH value:
The FE20 pH meter was calibrated using pH buffer solutions (pH 4.00, 6.86, and 9.18, respectively). The electrodes were rinsed with pure water, excess water was sucked off with fiber free paper, and then the electrodes were immersed in the liquid sample to be tested, followed by pressing the reading key to start the measuring. After the reading stabilized, the data obtained was the pH value of the sample.

If the pH value of the test solution was <5 or >9, the solution needed to be adjusted to pH 6~8 with acid or alkali. The commonly used pH regulators were NaOH and HCl, phosphoric acid and phosphate (e.g. sodium dihydrogen phosphate, disodium hydrogen phosphate), citric acid and citrate (e.g. sodium citrate), boric acid and borax; if the osmotic pressure of the obtained solution was detected not to reach an isotonic pressure, an appropriate amount of sodium chloride was added to make the solution reach or close to the isotonic.

Method for verifying the result of delivering medicaments to the posterior segment of the eye:
Experimental instrument and equipment: High performance liquid chromatography, model: LC-20AD (Shimadzu, Japan); mass spectrometer: model: API4000 triple quadrupole mass spectrometer (Applied Biosystems, USA); chromatographic column: Fortis Pace C18 5 µM, 2.1 × 30 mm (Fortis, UK).

Healthy adult Sprague Dawley (SD) rats were divided into a test group and a control group, with 6 eyes in each group. The test group received eye drops of the ophthalmic preparation prepared in the Example of the present invention, while the control group received the suspension of medicament (2 mg) in 5 mL of physiological saline (vortex shaking prior to use), at a dosage of 20 µL/eye. 0.5 h or 1 h after administration, the animals were euthanized, and the vitreous bodies were quickly collected. The vitreous samples were homogenized and stored at -80 °C. To 10 µL of vitreous homogenate, was added 90 µL of 95% ethanol, and then the solution was mixed under ultrasonic for 2 min, followed by vortex mixing for 1 min, to obtain the vitreous homogenate; to 50 µL of the homogenate, was added 175 µL of methanol, and then the resultant solution was vortex mixed for 3 min, and centrifuged at 4 °C and 12000 rpm for 10 min. The supernatant was filtered using a 0.45 µm needle filter. The filtrate was subjected to LC/MS/MS (Positive ion mode, MRM SCAN) analysis.

### Example 1. Preparing the ophthalmic preparation according to the present invention

According to Table 1, 0.24 g of CMC Na (sodium carboxymethyl cellulose, ionic polymer) was weighed and added to a glass triangular flask containing 40 mL of purified water, and then stirred for 2 h with a magnetic stirrer to obtain solution 1; 1.0 g of polysorbate 80 (surfactant) and 0.24 g of HPMC (hydroxypropyl methyl cellulose, adhensive agent) were respectively weighed and transferred into a glass triangular flask containing 60 mL of pure water, and then stirred with a magnetic stirrer and heated in an water bath at 40 °C for about 1.5 h, to obtain solution 2; 40 mg of dexamethasone and 4 mL of PEG400 (i.e. 4.3 times the amount of surfactant (w/w)) were added into solution 2, and then the resultant solution was further heated and stirred for 30 min, which was then added to solution 1, followed by stirring for 30 min, to obtain the mixed solution; the mixed solution was dispersed with a disperser for 5 min at a speed of 9500 rpm, the disperser was turned off, and the solution was rested until the foam disappeared, followed by filtering under reduced pressure with a Büchner funnel, to obtain the dispersed solution; the dispersed solution was transferred to a high-pressure homogenizer, and homogenized at the temperature of 15 ± 5 °C under the pressure of about 400 Bar for 3 min, then homogenized under the increased pressure of >800 Bar for 25 min, followed by under the reduced pressure of 300 Bar for 2 min. The solution was discharged to obtain a colorless and clear solution, which was further filtered under reduced pressure, so as to remove bacteria and mechanical impurities, and obtain a colorless and clear solution after removal of impurities.

Adjusting pH value and osmotic pressure: 700 mg of NaH₂PO₄ and 400 mg of Na₂HPO₄ were added to adjust pH = 6.3; sodium chloride was added to adjust osmotic pressure to be 282 mOsmol/kg.

HPLC detection: instrument Agilent 1100 high-performance liquid chromatography; operating software: OpenLab CDS c.01.10 (201) Chemstation Edition;

Chromatographic conditions: the chromatographic column was Waters XBridge C18 5 µm, 4.6 x 250 mm; column temperature 35 °C, flow rate 1.0 mL/min, detection wavelength 240 nm; mobile phase: 0.1% phosphoric acid aqueous solution (72.0%) - acetonitrile (28.0%) isocratic elution. The sample was diluted with the mobile phase in a ratio of 1:5, and then 10 µL of sample solution was injected into HPLC, with a test result of 96.2%.

The particle size was 20.6 nm (85.6%), and PdI was 0.266. The sample was stored at room temperature in dark for 1 month, and the appearance and the content was not changed.

### Example 2. Preparing the ophthalmic preparation according to the present invention

The preparation method was based on Example 1, and the raw materials and their amounts were shown in Table 1. After removal of impurities, a colorless and clear solution was obtained.

Adjusting pH value: 0.2 N NaOH and/or 0.1 N HCl was added to adjust pH to be 6.5.

The HPLC detection method was the same as that in Example 1. The content result detected by HPLC was 95.1%, the particle size was 12.9 nm (92.1%), PdI was 0.509; the stability was better, and there was no significant change in the appearance and the content after being stored in dark at room temperature for one month; a small amount of precipitation appeared after two months.

1 h after eye drops, the API concentration in the rat vitreum was 13.9 ng/g, with an RSD of 17.2%.

### Example 3. Preparing the ophthalmic preparation according to the present invention

The preparation method as well as the methods for adjusting pH value and osmotic pressure were based on Example 1. The raw materials and their amounts were shown in Table 1. After removal of impurities, a yellowish clear solution was obtained.

HPLC detection: Column ZORBAX Eclipse Plus C18, 4.6 x 100 mm 3.5 µm; mobile phase A: 0.1% phosphoric acid, mobile phase B: methanol (80:20) isocratic elution; Temp.: 35 °C, detection wavelength: 280 nm, flow rate: 0.8 ml/min; test result: 98.4%. Particle size 39.7 nm (95.5%), PdI: 0.318; after storing at room temperature in dark for 1 month, no changes in appearance or content was observed.

0.5 h after eye drops, the API concentration in the rat vitreum was 78.3 ng/g.

### Example 4. Preparing the ophthalmic preparation according to the present invention

The preparation method as well as the methods for adjusting pH value and osmotic pressure were based on Example 3. The raw materials and their amounts were shown in Table 1. After removal of impurities, a yellowish clear solution was obtained.

The HPLC detection method was the same as that in Example 3, and the detection result was 97.8%; particle size was 46.2 nm (95.5%); Pdl was 0.343; there was no significant change in appearance and content after being stored at room temperature in dark for one month.

### Example 5. Preparing the ophthalmic preparation according to the present invention

The preparation method was based on Example 1, and the raw materials and their amounts were shown in Table 1, wherein the amount of the cosolvent PEG400 was 5 times that of the surfactant (w/w), to obtain a colorless and clear solution after removal of impurities.

Adjusting pH value and osmotic pressure: 1M NaH₂PO₄ solution was added to adjust pH to be 6.2; sodium chloride was added to adjust osmotic pressure to be 295 mOsmol/kg;
the HPLC detection method was the same as that in Example 1, and the detection result was 97.3%; particle size was 22.4 nm (91.4%); Pdl was 0.293; there was no significant change in appearance and content after being stored at room temperature in dark for one month.
0.5 h after eye drops, the API concentration in the rat vitreum was 42.7 ng/g.

### Example 6. Preparing the ophthalmic preparation according to the present invention

The preparation method was based on Example 1, and the raw materials and their amounts were shown in Table 1, wherein the amount of the cosolvent PEG400 was 5 times that of the surfactant (w/w), to obtain a colorless and clear solution after removal of impurities.

pH value was 6.5, no adjustment required; referring to Example 1 for osmotic pressure regulation.

The HPLC detection wavelength was 245 nm, and the HPLC detection method was the same as that in Example 1, with a detection result of 98.1%;
the particle size was 18.6 nm (96.9%); Pdl was 0.257; there was no significant change in appearance and content after being stored at room temperature in dark for one month.
0.5 h after eye drops, the API concentration in the rat vitreum was 43.8 ng/g.

### Example 7. Preparing the ophthalmic preparation according to the present invention

The preparation method as well as the methods for adjusting pH value and osmotic pressure were based on Example 1. The raw materials and their amounts were shown in Table 1. After removal of impurities, a colorless clear solution was obtained.

The HPLC detection method was the same as that in Example 6, with a detection result of 98.3%;
the particle size was 18.5 nm (97.6%); Pdl was 0.208; there was no significant change in appearance and content after being stored at room temperature in dark for one month.

### Example 8. Preparing the ophthalmic preparation according to the present invention

The preparation method as well as the methods for adjusting pH value and osmotic pressure were based on Example 1. The raw materials and their amounts were shown in Table 1. After removal of impurities, a colorless clear solution was obtained.

The HPLC detection method was the same as that in Example 1, with a detection result of 96.8%;
the particle size was 18.7 nm (89.2%); Pdl was 0.255; there was no significant change in appearance and content after being stored at room temperature in dark for one month.

### Example 9. Preparing the ophthalmic preparation according to the present invention

The preparation method as well as the methods for adjusting pH value and osmotic pressure were based on Example 1, and the raw materials and their amounts were shown in Table 1, wherein the amount of the cosolvent PEG400 was 1 mL, i.e. 8 times that of the surfactant (w/w), to obtain a colorless and clear solution after removal of impurities.

The HPLC detection method was the same as that in Example 1, with a detection result of 97.2%;
the particle size was 19.6 nm (97.0%); Pdl was 0.289; there was no significant change in appearance and content after being stored at room temperature in dark for one month.

### Comparative example 1

The raw materials and their amounts were shown in Table 1. The surfactant was substituted with the povidone with low polymerization degree PVP12, and the ionic polymer with hydroxypropyl cellulose (HPC). The preparation method was based on Example 1, and 1 mL PEG400 was used as a cosolvent, to obtain a milky white solution.

Test results: the particle size was 899 nm (92.9%); Pdl was 0.188; white precipitates were formed when standing overnight.

### Comparative example 2

The raw materials and their amounts were shown in Table 1. The surfactant was substituted with the povidone with medium polymerization degree PVP30. The preparation method was based on Example 1, to obtain a colorless clear solution.

Test results: the particle size was 241 nm (53.1%) and 89.4 nm (32.6%); Pdl was 1.000; precipitates were formed after storing for one week.

### Comparative example 3

The raw materials and their amounts were shown in Table 1. The surfactant was substituted with the povidone with medium polymerization degree PVP30. The preparation method was based on Example 1, to obtain a colorless clear solution.

Test results: the particle size was 483 nm (47.6%) and 117 nm (33.9%); Pdl was 1.000; precipitates formed when storing for one week.

### Comparative example 4

The raw materials and their amounts were shown in Table 1, and the amounts of HPMC and CMC-Na were changed. The preparation method was based on Example 1, to obtain a white emulsion.

Test results: the particle size was 26.9 nm (40.5%) and 2043 nm (31.8%); Pdl was 1.000; white precipitates were observed when standing overnight.

### Comparative example 5

The raw materials and their amounts were shown in Table 1, and the amounts of HPMC and CMC-Na were changed. The preparation method was based on Example 1. 3 ml of PEG400 and 1 ml of ethanol were added as cosolvents, and then water was added to 100 mL, to obtain a colorless solution. Sodium chloride was added to adjust osmotic pressure to 262 mOsmol/kg.

Test results: the particle size was 416 nm (80%) and 18.1 nm (20%); Pdl was 0.462; the solution became a white emulsion after storing at room temperature for two weeks.

### Example 10. Preparing the ophthalmic preparation according to the present invention

The raw materials and their amounts were shown in Table 2. 0.52 g of povidone (PVP) K30 was weighed and added to a 100 mL glass triangular flask containing 25 mL of pure water, and then stirred for 2 h with a magnetic stirrer to obtain solution 1; 260 mg of HPMC and 420 mg of povidone (PVP) K12 were added to a 100 mL glass triangular flask containing 25 mL of pure water, and then stirred with a magnetic stirrer and heated in an water bath at 40 °C for 2 h, to obtain solution 2; 4 mL of PEG400 (cosolvent, its amount was 2.56 times that of PVP K12) and 20 mg of dexamethasone were added into solution 2, and then the resultant solution was further heated and stirred for 30 min, which was then added to solution 1, followed by stirring for 30 min, to obtain the mixed solution; using the operations of high-speed dispersion, high-pressure homogenization, and membrane filtration similar to Example 1, a colorless and clear solution was obtained after removal of impurities.

Adjusting pH and osmotic pressure: pH 6.5, no adjustment required; the osmotic pressure was adjusted with sodium chloride to 293 mmol/kg.

The HPLC detection method was the same as that in Example 1, with a detection result of 99.2%; the particle size was 575 nm (92.6%); Pdl was 0.211; there was no significant change in appearance and content after being stored at room temperature in dark for one month; the precipitation was observed after storing for two months.

0.5 h after eye drops, the API concentration in the rat vitreum was 5.1 ng/g.

### Example 11. Preparing the ophthalmic preparation according to the present invention

The raw materials and their amounts were shown in Table 2, the amount of cosolvent PEG400 was 8.9 times that of the surfactant (w/w). The preparation method as well as the methods for adjusting pH value and osmotic pressure were based on Example 10. A colorless and clear solution was obtained after removal of impurities.

The HPLC detection method was the same as that in Example 10, with a detection result of 98.5%;
the particle size was 125.6 nm (63.5%) and 13.6 nm (33.1%); Pdl was 0.255; there was no significant change in appearance and content after being stored at room temperature in dark for one month.

### Comparative example 6

The raw materials and their amounts were shown in Table 2, and the preparation method was based on Example 10. The ionic polymer was added to obtain light white emulsion.

Test results: the particle size was 1299 nm (92.4%); Pdl was 0.175; white precipitates formed when standing overnight.

### Comparative example 7

The raw materials and their amounts were shown in Table 2, and the preparation method was based on Example 10. 1 mL of PEG400 (20 times that of the surfactant (w/w)) was added as a cosolvent, to obtain a colorless solution.

Test results: the particle size was 637 nm (85.9%); Pdl was 0.258; white precipitates were formed when standing overnight.

### Comparative example 8

The raw materials and their amounts were shown in Table 2, and the preparation method was based on Example 10. Without povidone, the ionic polymer was added, to obtain a colorless solution.

Test results: pH 5.2; the particle size was 46.2 nm (96.9%); Pdl was 0.343; the content was 98.9% detected by HPLC; fine crystals precipitated after storing for 2 weeks in a refrigerator.

After eye drop administration, the test results of API concentration in the vitreous body of rats indicated that the ophthalmic preparation of the present invention could carry the active pharmaceutical ingredients for treating eye diseases and cross the barrier of the eyeball structure, and thereby deliver the effective dose of medicaments to the vitreous body by means of conjunctival sac administration (eye drop administration), which could avoid invasive administration methods such as intravitreal injection, and also significantly reduce the total drug amount and the absorption of drugs in the whole body, so as to prevent toxic and side effects.

**Table 1.**

| Example | Surfactant (A) | Ionic polymer (B) | Adhesive agent (C) | Amount of API (D) (mg) | Mass ratio of A:B:C:D | Cosolvent | Solve nt |
|---|---|---|---|---|---|---|---|
| 1 | Polysorbate 80 | Sodium carboxymethyl cellulose (CMC-Na) | Hydroxypropyl methylcellul ose (HPMC) | Dexamethasone | 25:6:6:1 | PEG400 | Water 100 mL |
| | | | | 40 mg | | | |
| 2 | Polysorbate 20 | CMC-Na | Carbomer 941 | Dexamethasone | 30:5:1:1 | | Water 25 mL |
| | | | | 10 mg | | | |
| 3 | Poly-sorbate 80 | CMC-Na | PVPK12 | Diclofenac | 12:4:6:1 | | Water 30 mL |
| | | | | 30 mg | | | |
| 4 | Polysorba te 80 | Sodium hyaluronate | HPMC | Diclofenac | 12:2:6:1 | | Water 30 mL |
| | | | | 30 mg | | | |
| 5 | Span 40 | CMC-Na | HPMC | Dexamethaso ne | 22:5:6:1 | PEG400 | Water 25 mL |
| | | | | 10 mg | | | |
| 6 | Polysorba te 80 | CMC-Na | Poloxamer P407 | Hydrocortiso ne | 25:6:5:1 | PEG400 | Water 10 mL |
| | | | | 4 mg | | | |
| 7 | Polysorba te 80 | CMC-Na | HPMC | Hydrocortiso ne | 25:6:5:1 | | Water 100 mL |
| | | | | 40 mg | | | |
| 8 | Polysorba te 80 | CMC-Na | Poloxamer P188 | Prednisolone | 25:6:5:1 | PEG300 | Water 10 mL |
| | | | | 4 mg | | | |
| 9 | Polysorba te 80 | CMC-Na | HPMC | Betamethaso ne | 31:7.5:7.5 :1 | PEG400 | Water 10 mL |
| | | | | 4 mg | | | |
| Comparat ive example 1 | PVP K12 | HPC | HPMC | Dexamethaso ne | 20:10:10: 1 | PEG400 | Water 25 mL |
| | | | | 10 mg | | | |
| Comparat ive example 2 | PVP K30 | CMC-Na | HPC | Hydrocortiso ne | 25:6:5:1 | | Water 10 mL |
| | | | | 4 mg | | | |
| Comparat ive example 3 | PVP K30 | CMC-Na | HPC | Prednisolone | 25:6:5:1 | | Water 10 mL |
| | | | | 4 mg | | | |
| Comparat ive example 4 | Polysorba te 80 | HPMC | CMC-Na | Prednisolone | 25:6:5:1 | | Water 10 mL |
| | | | | 4 mg | | | |
| Comparati ve example 5 | Polysorba te 80 | HPMC | CMC-Na | Dexamethaso ne | 20:10:1:1 | 3 mL PEG400 1 mL ethanol | Water 100 mL |
| | | | | 40 mg | | | |

**Table 2**

| Exampl e | Povidone with low polymerizati on degree (A) | Povidone with medium polymerizati on degree (B) | Adhesive agent (C) | API(D) | Mass ratio of A:B:C:D | Cosolv ent | Solve nt in total volum e |
|---|---|---|---|---|---|---|---|
| 10 | PVP K12 | PVP K30 | HPMC | Dexamethason e | 21:26:13:1 | PEG40 0 | Water 50 mL |
| | | | | 20 mg | | | |
| 11 | PVP K12 | PVP K30 | HPMC | Prednisolone | 6:25:5:1 | PEG40 0 (0.2m L) | Water 10 mL |
| | | | | 4 mg | | | |
| Compar ative example 6 | PVP K12 | CMC-Na | PVP K30 | Dexamethason e | 15:15:5:1 | | Water 25mL |
| | | | | 10 mg | | | |
| Comparative example 7 | PVP K12 | PVP K30 | PVP K30 | Dexamethason e | 15:7.5:7.5:1 | PEG 400 | Water 25 mL |
| | | | | 10 mg | | | |
| Compar ative example 8 | N/A | CMC-Na | HPMC | Diclofenac | 0:3:4:1 | | Water 30 mL |
| | | | | 30mg | | | |

In the following, the beneficial effects of the pharmaceutical preparations of the present invention for eye drops were demonstrated with reference to the Experimental examples.

### Experimental example 1: Observation results of the carriers according to the present invention by transmission electron microscope

Transmission Electron Microscope (JEM-2100 Plus, JEOL, Japan)

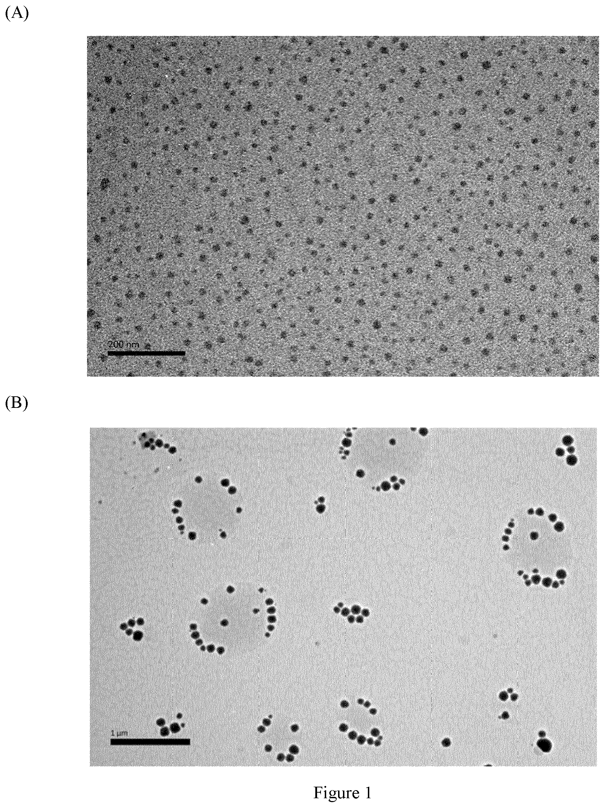

One drop of the liquid sample prepared in Example 1 was transferred into the copper mesh, and after standing for 5 min, any excess liquid sample was removed, then the copper mesh was allowed to dry naturally and placed in the specimen chamber for testing; sample staining: one drop of liquid sample was transferred into the copper mesh, and after removing the excess sample from the sample mesh, one drop of 2% phosphomolybdic acid was added, followed by standing for 5 min. Then, the excess liquid was removed, and the mesh was naturally dried, and placed in the electron microscope for testing. The results are shown in Figure 1. It could be found that the drug carrier prepared in the present invention formed a spherical structure with a particle size of 1-100 nm in the solvent (nanobodies, Figure 1A), and the nanobodies can further self-assemble into spheres with a particle size of 10-2000 nm (nanospheres, Figure 1B).

### Experimental example 2: Detection of the particle size, the content, and the stability

### 1. Experimental methods

1 mL of sample prepared in the Example and the Comparative example was transferred to the sample cell, and the detection temperature was set to 40 °C. The sample cell was put into NS-90 nanoparticle size analyser for testing. Each sample was tested three times, and the average of the three test results was represented by the particle size (in terms of light intensity distribution and % percentage) and the polydispersity index (PdI). After testing, the sample was stored in dark, the appearance changes were observed, and then the particle size was retested.

The content of the ophthalmic preparation sample prepared in the present invention was detected by Agilent 1100 high-performance liquid chromatography.

### 2. Experimental results

See Table 3.

**Table 3.**

| Exam ple | Initial particle size (percentage)/PdI/ HPLC content | Storing in a dark place at a certain temperature for one month | |
|---|---|---|---|
| | | Particle size (percentage)/PdI | Appearance and content changes |
| 1 | 20.6 nm (85.6%), PdI: 0.266 HPLC content: 96.2% | room temperature, 20.2 nm (89.3%), 0.247 | No significant changes were observed. |
| 2 | 12.9 nm (92.1%), PdI: 0.509 HPLC content: 95.1% | room temperature, 14.7 nm (86.2%), 0.325 | No significant changes were observed after one month; (Precipitation was observed after two months.) |
| 3 | 39.7 nm (95.5%), PdI: 0.318 HPLC content: 98.4% | room temperature, 40.2 nm (92.7%), PdI: 0.258 | No significant changes were observed. |
| 4 | 46.2 nm (95.5%), PdI: 0.343 HPLC content: 97.8% | room temperature, 43.8 nm (91.4%), PdI: 0.226 | No significant changes were observed. |
| 5 | 22.4 nm (91.4%), PdI: 0.293 HPLC content: 97.3% | room temperature, 20.3 nm (95.2%), PdI: 0.240 | No significant changes were observed. |
| 6 | 18.6 nm (96.9%), PdI: 0.257 HPLC content: 98.1% | room temperature, 19.3 nm (95.2%), PdI: 0.226 | No significant changes were observed. |
| 7 | 18.5 nm (97.6%), PdI: 0.205 HPLC content: 98.3% | room temperature, 19.3 nm (95.2%), PdI: 0.226 | No significant changes were observed. |
| 8 | 18.7 nm (89.2%), PdI: 0.255 HPLC content: 96.8% | room temperature, 19.3 nm (92.0%), PdI: 0.306 | No significant changes were observed. |
| 9 | 19.6 nm (97.0%),PdI:0.289 HPLC content: 97.2% | room temperature; 21.4 nm (93.5%), PdI: 0.269 | No significant changes observed. |
| 10 | 575 nm (92.6%), PdI: 0.211 HPLC content: 99.2% | room temperature, 216 nm (94.1%), PdI: 0.368 | No significant changes were observed after one month; precipitation was observed after two months. |
| 11 | 125.6 nm (63.5%) and 13.6 nm (33.1%), PdI: 0.255 HPLC content: 98.5% | room temperature, 26.3 nm (89.2%), PdI: 0.301; particle size distribution became narrow. | No significant changes were observed. |
| Comp arative examp le 1 | 899 nm (92.9%), PdI: 0.188 | Standing overnight at room temperature. | White precipitate appeared. |
| Comp arative examp le 2 | 241 nm (53.1%) and 89.4 nm (32.6%), PdI: 1.000 | Storing at room temperature for one week. | White precipitate appeared. |
| Comp arative examp le 3 | 483 nm (47.6%) and 117 nm (33.9%), PdI: 1.000 | Storing at room temperature for one week. | White precipitate appeared. |
| Comp arative examp le 4 | 26.9 nm (40.5%) and 2043 nm (31.8%), PdI: 1.000 | Standing overnight at room temperature. | Becoming white emulsion. |
| Comp arative examp le 5 | 416 nm (80%) and 18.1 nm (20%), PdI: 0.462, | Storing at room temperature for two weeks. | Becoming white emulsion. |
| Comp arative examp le 6 | 1299 nm (92.4%), PdI: 0.175 | Standing overnight at room temperature | White precipitate appeared. |
| Comp arative examp le 7 | 637 nm (85.9%), PdI: 0.258 | Standing overnight at room temperature | White precipitate appeared. |
| Comp arative examp le 8 | 46.2 nm (96.9%), PdI: 0.343 HPLC content: 98.9% | Storing for 2 weeks in a refrigerator | Crystalline precipitation. |

The above results indicated that the pharmaceutical preparation prepared in the present invention had a small particle size, a high content of active pharmaceutical ingredients detected by HPLC, as well as the stable morphology and content after long-term storage; this indicated that the preparation of the present invention had high encapsulation efficiency and good stability. However, the preparation prepared in the Comparative example using different excipients and starting materials from the present invention had poor stability and might experience precipitation or change in a short period of time.

### Experimental example 3. Verifying the effect of the ophthalmic preparation according to the present invention on macular edema after eye drops

### 1. Experimental methods

48 SD rats with no obvious abnormalities in both eyes were randomly divided into 6 groups (8 rats in each group, half male and half female). The photosensitizer Rose Bengal (40 mg/mL, 40 mg/kg) was injected into the tail vein on D1, and then the rats immediately underwent laser photocoagulation of a single retinal vein in the right eye. Approximate 15 minutes after completion of the photocoagulation, it was confirmed whether the target retinal vein was blocked. After confirming the successful obstruction, the testing substance was administered according to Table 4. The testing substance originated from the preparation prepared in Example 1 was used in the high-dose group, the preparation prepared in Example 1 was diluted with medical physiological saline in a ratio of 1:3 to obtain the medium dose, and the preparation prepared in Example 1 was diluted with medical physiological saline in a ratio of 1:9 to obtain the low dose; the commercially available drugs aflibercept intraocular injection (Bayer, Germany) and dexamethasone sodium phosphate injection (Sinopharm Ronshyn Pharmaceutical Co., Ltd., China) were used as control drugs. Fundus photography (FP), fundus fluorescein angiography (FFA), and optical coherence tomography (OCT, without testing immediately after D1 molding) were performed on the right eye of all animals on D-1 (before modeling), D1 (immediately after modeling), D2 (on the second day after modeling), and D4 (on the fourth day after modeling). Image analysis was performed on FP and FFA images, and the full thickness of the retina at approximate 750 µm and 1500 µm on the left and right sides of the obstructed vein was measured on the OCT scan. The left eyes of all animals had not underwent modeling, administration, or corresponding ophthalmic examinations.

**Table 4. Animal grouping and administration table**

| Group | | Administration | | | | | Animal number |
|---|---|---|---|---|---|---|---|
| | | Testing solution/ Control drug | Time and frequency | Concentration (mg/mL) | Volume (µL) | Administration pathways | |
| 1 | Medium control group | Physiological saline | D1-D3, 4 times/day; D4, once | - | 20 | Conjunctival sac eye drops | 8 |
| 2 | Test substance | Low dose group | | 0.044 | 20 | | 8 |
| 3 | Test substance | Medium dose group | | 0.133 | 20 | | 8 |
| 4 | Test substance | High dose group | | 0.4 | 20 | | 8 |
| 5 | Commercially available control group 1 | Aflibercept intraocular injection | D1, once | 40 | 5 | Intravitreal injection | 8 |
| 6 | Commercially available control group 2 | Dexamethasone sodium phosphate injection | | 0.6 | 5 | | 8 |

### 2. Experimental results

As shown in Figure 2, it could be found that there was a significant increase in the thickness of the retina after modeling, while a significant decrease in the thickness of the retina could be observed on D2 and D4 after eye drops of the preparation according to the present invention. Moreover, its effect was equivalent to that of intravitreal injection of commercially available drugs, indicating that the preparation of the present invention could achieve a very good effect on improving retinal edema by eye drops.

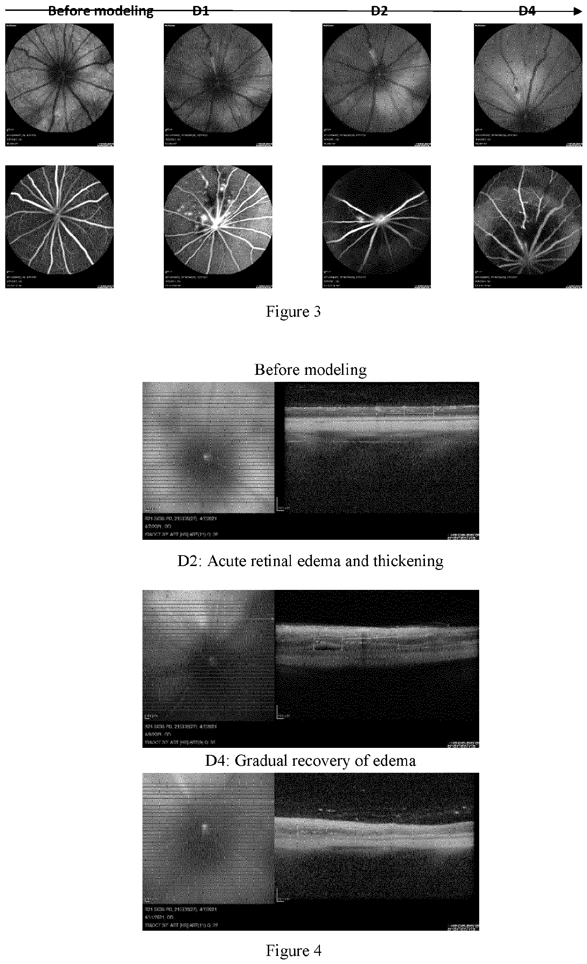

As shown in Figure 3, after modeling (D1), there was a significant venous thrombosis as well as stasis of blood stream at the laser photocoagulation site compared to before modeling; After administering the preparation of the present invention by eye drops, although there was still some retinal vein obstruction, venous thrombosis has been significantly improved.

As shown in Figure 4, acute retinal edema and thickening occurred on D2 after administrating the preparation of the present invention, but gradually recovered on D4, close to the normal state before modeling.

The above results indicated that for the medium dose (0.133 mg/mL) and the high dose (0.4 mg/mL) of the test substance, repeated administration by eye drops (20 µL/eye, for 4 days, 4 times/day in the first 3 days, while single dose on the 4th day) had a significant inhibitory/therapeutic effect on acute retinal edema induced by laser photocoagulation combined with photosensitizers in rats with retinal vein occlusion, and there was a dose-dependent effect. The high-dose group presented a similar efficacy to the commercially available control drug Aflibercept intraocular injection. In this experiment, the commercially available control dexamethasone sodium phosphate injection did not show a clear inhibitory/therapeutic effect on acute retinal edema after one-time vitreous injection.

In summary, the present invention provided a preparation by eye drop administration, which, by eye drop administration, could carry (encapsulate) glucocorticoids and/or non-steroidal drugs through the anterior segment and deliver them to the posterior segment of the eye, to produce therapeutic effects. The present invention had achieved the goal of treating macular edema and other fundus diseases by eye drops, solved the technical problems that urgently needed to be solved but still had not been solved in this field, and thereby had extremely excellent clinical value and very positive social significance.

## Claims

1. An ophthalmic preparation for eye drops, **characterized in that** it is a preparation composed of an active pharmaceutical ingredient for treating eye diseases and an ophthalmic preparation carrier or auxiliary material;
the active ingredient for treating eye diseases is a glucocorticoid medicine and/or a non-steroidal medicine;
the ophthalmic preparation carrier or auxiliary material comprises the following ingredients: a surfactant, an ionic polymer and a solvent;
or, the ophthalmic preparation carrier or auxiliary material contains the following ingredients: povidone with low polymerization degree, povidone with medium polymerization degree and a solvent.

2. The preparation according to claim 1, **characterized in that** in the ophthalmic preparation carrier or auxiliary material, the mass ratio of the surfactants to the ionic polymer is: (1-100):(0.1-50); the ratio of the surfactant to the solvent is: every 100 mL of the solvent contains 5-3000 mg of the surfactant.

3. The preparation according to claim 2, **characterized in that** in the ophthalmic preparation carrier or auxiliary material, the mass ratio of the surfactants to the ionic polymer is (12-31):(2-7.5); the ratio of the surfactant to the solvent is: every 100 mL of the solvent contains 880-1240 mg of the surfactant.

4. The preparation according to any one of claims 1 to 3, **characterized in that** the surfactant is a non-ionic surfactant; preferably, the non-ionic surfactant is Spans, Polysorbates, Poloxamer, alkylglucosides, vitamin E polyethylene glycol succinate (TPGS), sucrose stearates or azone; Spans or polysorbates are preferable.

5. The preparation according to any one of claims 1 to 3, **characterized in that** the ionic polymer is selected from at least one of carboxymethyl cellulose (CMC) and its salts, sodium starch glycolate, hyaluronic acid and its salts, Xanthan gum, alginic acid and its salts, and polyethylene glycol diacetate PEG-(COOH)₂; preferably, the ionic polymer is selected from at least one of carboxymethyl cellulose and its salts, hyaluronic acid and its salts.

6. The preparation according to claim 1, **characterized in that** in the ophthalmic preparation carrier or auxiliary material, the mass ratio of the povidone with low polymerization degree to the povidone with medium polymerization degree is (0.1-10):1, and the ratio of the povidone with low polymerization degree to the solvent is: every 100 mL of solvent contains 5-3000 mg of the povidone with low polymerization degree;
preferably, the mass ratio of the povidone with low polymerization degree to the povidone with medium polymerization degree is (0.24-0.8):1, and the ratio of the povidone with low polymerization degree to the solvent is: every 100 mL of solvent contains 240-840 mg of the povidone with low polymerization degree.

7. The preparation according to claim 6, **characterized in that** the povidone with low polymerization degree is a povidone with a weight average molecular weight of 2000-5000, and the povidone with medium polymerization degree is a povidone with a weight average molecular weight of 20000-60000.

8. The preparation according to claim 6, **characterized in that** the povidone with low polymerization degree is a povidone PVP K12 with a weight average molecular weight of 3500, and the povidone with medium polymerization degree is a povidone PVP K30 with a weight average molecular weight of 35000-50000.

9. The preparation according to any one of claims 1 to 8, **characterized in that** in the ophthalmic preparation carrier or auxiliary material, the solvent is a polar solvent, and preferably water.

10. The preparation according to any one of claims 1 to 8, **characterized in that** the ophthalmic preparation carrier or auxiliary material also contains the following components: adhesive agents and/or cosolvents;
preferably, the adhesive agent is selected from at least one of polyethylene glycol, carbomer, Poloxamer, povidone, hydroxyethyl cellulose, hydroxypropyl cellulose, methyl cellulose, polyvinyl alcohol, Xanthan gum, polyoxyethylene fatty alcohols, hyaluronic acid and its salts or hydroxypropyl methyl cellulose (HPMC); the cosolvent is propylene glycol, glycerol, liquid polyethylene glycol or castor oil; the mass ratio of the adhesive agent to the surfactant is 1:(0.1-100), and the mass ratio of the cosolvent to the surfactant is (1-10):1; the mass ratio of the adhesive agent to the povidone with low polymerization degree is 1:(0.1-100), and the mass ratio of the cosolvent to the povidone with low polymerization degree is (1-10):1;
more preferably, the mass ratio of the adhesive agent to the surfactant is 1:(1.2-30), and the mass ratio of the cosolvent to the surfactant is (2.56-9):1; the mass ratio of the adhesive agent to the povidone with low polymerization degree is 1:(1.2-30), and the mass ratio of the cosolvent to the povidone with low polymerization degree is (2.56-9):1.

11. The preparation according to claim 1, **characterized in that** the mass ratio of the surfactant or the povidone with low polymerization degree to the active pharmaceutical ingredient for treating eye diseases is (12-31):1.

12. The preparation according to claim 1, **characterized in that** the glucocorticoid medicament is at least one of dexamethasone, hydrocortisone, prednisolone and betamethasone; the non-steroidal medicament is at least one of diclofenac, pranoprofen, indomethacin and bromfenac sodium.

13. The preparation according to any one of claims 1 to 12, **characterized in that** the ophthalmic preparation carrier or auxiliary material contains nanobodies, which are formed by self-assembly of the components of the ophthalmic preparation carrier or auxiliary material; the nanobodies are assembled with active pharmaceutical ingredients for treating eye diseases.

14. The preparation according to claim 13, **characterized in that** the nanobody is spherical, with a particle size of 1-100 nm; preferably, the particle size of the nanobody is 5-30 nm.

15. The preparation according to claim 14, **characterized in that** it contains nanospheres, which are spherical in shape and have a particle size of 10-2000 nm; the nanospheres are formed by self-assembly of nanobodies; preferably, the particle size of the nanospheres is 100-2000 nm.

16. A method for preparing the preparation according to any one of claims 1 to 15, **characterized in that** it comprises the following steps:
(1) The surfactant and/or the adhesive agent is added to the solvent to prepare a solution;
(2) The active pharmaceutical ingredient for treating eye diseases and/or the cosolvent is dispersed in the solution obtained in step (1), to which is then added the ionic polymer or its solution, and the resultant solution is dispersed and mixed to obtain the initial suspension;
(3) The initial suspension obtained in step (2) is stirred and dispersed or homogenized to obtain the preparation;
or comprises the following steps:
(a) The povidone with low polymerization degree and/or the adhesive agent is added to the solvent to prepare a solution;
(b) The active pharmaceutical ingredient for treating eye diseases and/or the cosolvent is dispersed in the solution obtained in step (a), to which is then added the povidone with medium polymerization degree or its solution, and the resultant solution is dispersed and mixed to obtain the initial suspension;
(c) The mixed solution obtained in step (b) is ground or uniformly dispersed to obtain the preparation.

17. The method according to claim 16, **characterized in that** the dispersion described in step (2) or step (b) is selected from at least one of mechanical stirring dispersion, magnetic stirring dispersion, vortex shaking dispersion, shear dispersion, homogeneous dispersion, grinding dispersion, and ultrasonic dispersion.

18. The preparation according to any one of claims 1 to 15 for use in the preparation of medicaments for treating fundus diseases; preferably, the medicament for treating fundus diseases is a medicament for treating macular edema, and/or optic neuritis, and/or non-infectious endophthalmitis.

19. The use according to claim 18, **characterized in that** the medicament for treating macular edema is a medicament for treating macular edema caused by fundus vascular diseases, macular edema caused by central retinal vein occlusion, macular edema caused by branch retinal vein occlusion, diabetic macular edema (DME), pathological myopic macular edema, and/or macular edema caused by wet age-related macular degeneration.

20. A method for treating fundus diseases, **characterized in that** the preparation according to any one of claims 1 to 15 is administrated to the patients at an effective therapeutic amount.

21. The method according to claim 20, **characterized in that** the fundus disease is macular edema, and/or optic neuritis, and/or non-infectious endophthalmitis.

22. The method according to claim 21, **characterized in that** the macular edema is macular edema caused by fundus vascular diseases, macular edema caused by central retinal vein occlusion, macular edema caused by branch retinal vein occlusion, diabetic macular edema (DME), pathological myopic macular edema, and/or macular edema caused by wet age-related macular degeneration.

23. The method according to claim 20, **characterized in that** the way used is eye drop administration.
